# EUROPEAN PATENT SPECIFICATION

(11) **EP 1 945 236 B1**
(45) Date of publication and mention of the grant of the patent: **01.04.2009**
(21) Application number: 06809008.3
(22) Date of filing: 10.10.2006
(51) Int. Cl.: A61K 36/28, A61P 1/02, A61K 9/00

(54) **A COMPOSITION FOR THE TREATMENT OF DIABETIC PERIODONTITIS**
ZUSAMMENSETZUNG ZUR BEHANDLUNG VON DIABETISCHER PERIODONTITIS
COMPOSITION POUR LE TRAITEMENT DE LA PARODONTITE DIABETIQUE

(30) Priority: 14.10.2005 HU 0500948
(43) Date of publication of application: 23.07.2008
(73) Proprietor: Mendon Trade & Commerce LC, Wilmington DE 19801 (US)
(72) Inventor: LITERÁTI NAGY, Péter, H-1037 Budapest (HU); LOHINAI, Zsolt, H-1161 Budapest (HU); TORY, Kálmán, H-1137 Budapest (HU); KOLONICS, Attila, H-1141 Budapest (HU); KÉRI, Ágnes, H-1015 Budapest (HU); RIGÓ, Orsolya, H-2083 Solymár (HU); HUSZÁK, András, H-1125 Budapest (HU); ZÁHONYI, Balázs, H-1119 Budapest (HU); BERNÁTH, Sándor, H-2089 Telki (HU); VIGH, László, H-6726 Szeged (HU); BODNÁR, Tibor, H-1171 Budapest (HU); EGRI, János, H-1036 Budapest (HU)
(74) Representative: Beszédes, Stephan G.
(86) International application number: PCT/IB2006/002856
(87) International publication number: WO 2007/042925

(56) References cited:
- DATABASE WPI Week 199235 Derwent Publications Ltd., London, GB; AN 1992-286776 XP002438015 & HU 59 815 A (BIOGAL GYOGYSZERGYAR) 28 July 1992 (1992-07-28) cited in the application

## Description

### Field of the invention

The invention refers to a use of an extract of a part of a goldenrod (Solidago) species, wherein said part has grown above the earth, or the solid residue remaining after the removal of the solvent content of the extract as the active agent for the preparation of a pharmaceutical composition suitable for the treatment of diabetic periodontitis.

### Background of the invention

Out of the diseases that occur in the oral cavity, gingivitis and periodontitis (i.e. periodontal disease) are extremely widespread, consequently, they can be considered as pandemic [Jenkins, W. M. és Papapanou, P.N.: Epidemiology of periodontal disease in children and adolescents, Periodontology 2000, 26, 16-32 (2001)]. Gingivitis occurs in 70 to 90 % of the population including children. In case of gingivitis, the gum (i.e. gingiva) is swollen, red and bleed easily. Untreated gingivitis subsisting permanently may lead to the formation of periodontitis.

Periodontitis extends to more than two thirds of the population [Murray, J.J.: The Prevention of Dental Disease, Oxford University Press, 1988, Chapter 10: The prevention and control of chronic periodontal disease, 328-372]. Periodontitis is a disease of the tissues that support and attach the teeth and having different stages depending on the severity of the disease. Untreated periodontitis results in the formation of gaps between the gums and the teeth (i.e. periodontal pockets), the loss of periodontal ligaments that attach the tooth to the jaw, the loss of alveolar bone and, lastly, tooth loss. Above 30 of age, the number of tooth lost because of periodontitis is growing increasingly. Periodontitis can be extended to the whole set of teeth (generalized periodontitis) or it can occur locally (localized periodontitis or inflammation of the periodontal pocket). Significant risk factors of the formation of periodontitis include age, environmental factors (for example smoking) and various systemic diseases [Mealey, B.L: Periodontal Implications: Medically Compromised Patients, Ann. Periodontol., 1, 256-321 (1996)].

Serious periodontitis occurs significantly more frequently among patients suffering from diabetes than within the average population [Axelsson, P.: Diagnosis and Risk Prediction of Periodontal Diseases, Quintessence Publishing Co., Inc., 2002, p. 175]. Periodontitis is a frequent complication of diabetes and it is probably connected with the chronic inflammation, reduced bone formation and micro-circulation disorder that reduces tissue regeneration, all of which are developped by diabetes. [Kathryn, E. et al.: Inflammation, stress, and diabetes, J. Clin. Invest., 115, 1111-1119 (2005); Hongbing, H. et al.: Diabetes causes decreased osteoclasto-genesis, reduced bone formation and enhanced apoptosis of osteablastic cells in bacteria stimulated bone loss, Endocrinology, 145, 447 (2005); McMullen, J.A. et al.: Microangiopathy within the gingival tissues of diabetic subjects with special reference to the prediabetic state, Periodontics, 5, 61-69 (1967)]. The diabetic periodontitis is a grave problem especially in case of type 1 diabetes mellitus (IDDM) that occurs especially in young age since the patient can loose the teeth even before reaching thirty of age. However, the interaction between diabetes and periodorititis has two directions: in addition to the fact that the bad metabolic state that is typical in diabetes predisposes the patient to periodontitis, the consequence of this latter is a worsened metabolic equilibrium [Losche, W. et al.: Plasma lipid and blood glucose levels in patients with destructive periodontal disease, J. Clin. Periodontal., 2000 Aug. 27 (8), 537-41].

Mouth washes, tooth pastes or tooth gels containing medicinal herb extracts are widely employed for the treatment of gingivitis. For example, DE-P No. 19 59 275 describes a mouth care composition containing camomile extract to achieve a reduction of the inflammation in the oral cavity. HU-P 207 792 describes a tooth paste and mouth wash containing extracts of Plantago lanceolata and Solidago canadensis in a mass ratio of 1:5 - 5:1 and medicinal water having a pH value of 7.0-8.2 in addition to conventional carriers for the reduction of mouth inflammation and gingivitis.

In case of mouth and tooth care compositions containing extracts of medicinal herbs, of course, the aim is the reduction of gingivitis, bleeding of gum (i.e. gingiva) and tartar formation. Periodontitis is a much more serious state that could be aimed at by a mouth and tooth care composition. Several scientific publications evaluate the effect of extracts of medicinal herbs. Pistorius, A. et al. examined a mouth wash containing extracts of medicinal herbs (Salvia offcinalis, Mentha piperia, menthol, camomile, Commiphora myrrha, Carvum carvi, Eugenia caryophyllus and Echinacea purpurea) in an irrigation treatment lasting for 3 months. At the end of the study bleeding of the gum was reduced, however, the depth of the inflammated periodontal pocket did not diminish [Pistorius, A. et al.: Efficacy of Subgingival Irrigation Using Herbal Extracts on Gingival Inflammation, J. Periodontol., 74, 616-622 (2003)]. However, van der Weijden, G.A. et al. examined a mouth wash containing the extract of three medicinal herbs (Juniperus communis, Urtica dioca and Achillaea millefolium) and did not find any influence on the tartar formation and the state of gum [Van der Weijden, G.A. et al.: The effect of herbal extracts in an experimental mouth rinse on established plaque and gingivitis, J. Clin. Periodontol., 25, 399-403 (1998)]. Chan, Y et al. examined the effect of Chinese medicines based on medicinal herbs on the periodontitis in animals stating that in the treatment of the experimentally induced periodontitis no significant difference between the treated animals and the control ones was found regarding inflammation, alveolar bone resorption and improvement of periodontitis [Chan, Y. et al: The evaulation of Chinese herbal medicine effectiveness on periodontal pathogens, Am. J. Chin. Med., 31(5), 751-61 (2003)]. Thus, it is evident that the known mouth and tooth care compositions containing extracts of medicinal herbs can reduce gingivitis or prevent the formation thereof at best, but they are not suitable for the treatment of an existing periodontitis.

In the clinical practice during treatment of diabetic periodontitis, at first, the wrong hygienic state of mouth is improved by treatment with bactericide agents and removal of the tartar. After or parallel with this treatment the periodontitis itself is treated with an antibiotic e.g. doxycycline. However, antibiotic treatment, especially when it is carried out for a long time, has unfavourable side-effects and patients, in general, wish to avoid such side-effects.

The aim of the invention is the preparation of a composition that is other than an antibiotic, practically without unfavourable side-effects and suitable for the effective treatment of diabetic periodontitis.

### Summary of the invention

It has been found that a pharmaceutical composition prepared from an extract of a part of a goldenrod (Solidago) species, wherein said part has grown above the earth, or the solid residue remaining after the removal of the solvent content of the extract as the active agent can be used for the effective treatment of diabetic periodontitis.

This fact is surprising for an expert since, based on the above results of the studies carried out on compositions containing extracts of medicinal herbs, the expert does not think of treating periodontitis, particularly diabetic periodontitis, with an extract of a medicinal herb.

Thus, the invention consists in the use of an extract of a part of a Solidago species, wherein said part has grown above the earth, or the solid residue remaining after the removal of the solvent content of the extract as the active agent for the preparation of a pharmaceutical composition suitable for the treatment of diabetic periodontitis.

### Description of preferred embodiments

Under the expression a "Solidago species" mainly the following medicinal plants are meant in terms of the taxonomical description:
**Class:** Magnoliopsida
**Subclass:** Asteridae
**Family:** Asteraceae
**Genus:** Solidago L.
**Species:**
   Solidago alpestris
   S. alpicola
   S. cambrica
   S. canadensis
   S. gigantea
   S. gigantea ssp. serotina
   Solidago graminifolia (L.) Salisb.
   S. Hartmanniana
   Solidago ×hirtipes Fern
   S. Horvatii
   S. jailarum
   S. lapponica
   S. longifolia
   S. macrhorriza
   S. maritima
   S. minuta
   S. monicola
   Solidago x Niederederi
   S. scepusiensis
   Solidago pauciflosculosa
   S. Pritcheri
   S. serotina
   S. Shortii
   S. taurica
   S. valesiaca
   S. virgaurea
   S. virgaurea ssp. alpestris
   S. virgaurea ssp. macrorrhiza
   S. virgaurea ssp. Minuta
   S. virgaurea ssp. vulgaris
   S. vulgaris
   Solidago arguta Ait. ssp. caroliniana (Gray) G. Morton
   Solidago arguta Ait. ssp. pseudoyadkinensis G. Morton
   Solidago boottii Hook. var. caroliniana (Gray) Cronq.
   Solidago yadkinensis (Porter) Small
   Solidago arguta Ait. var. harrisii (Steele) Cronq.
   Solidago harrisii Steele
   Solidago arguta Ait. var. neurolepis (Fern.) Steyermark
   Solidago neurolepis Fern.
   Solidago ×asperula Desf. (pro sp.) [rugosa × sempervirens]
   Solidago auriculata Shuttlw. ex Blake
   Solidago amplexicaulis Torr. & Gray ex Gray, non Martens
   Solidago notabilis Mackenzie
   Solidago ×beaudryi Boivin [rugosa × uliginosa]
   Solidago bicolor L.
   Solidago brachyphylla Chapman
   Solidago boottii Hook. var. brachyphylla (Chapman) Gray
   Solidago buckleyi Torr. & Gray
   Solidago caesia L.
   Solidago caesia L. var. caesia
   Solidago axillaris Pursh
   Solidago caesia L. var. axillaris (Pursh) Gray
   Solidago caesia L. Var. curtisii (Torr. & Gray) Wood
   Solidago caesia L. var. hispida Wood
   Solidago curtisii Torr. & Gray
   Solidago curtisii Torr. & Gray var. pubens (M.A. Curtis) Gray
   Solidago lancifolia Torr. & Gray
   Solidago monticola Torr. & Gray
   Solidago pubens M.A. Curtis
   Solidago calcicola Fern.
   Solidago californica Nutt.
   Solidago canadensis L.
   Solidago canadensis L. var. canadensis
   Solidago canadensis L. var. gilvocanescens Rydb.
   Solidago altissima L. var. gilvocanescens (Rydb.) Semple
   Solidago gilvocanescens (Rydb.) Smyth
   Solidago pruinosa Greene
   Solidago canadensis L. var. hargeri Fern.
   Solidago canadensis L. var. lepida (DC.) Cronq.
   Solidago canadensis L. var. subserrata (DC.) Cronq. Solidago lepida DC.
   Solidago lepida DC. var. molina Fern.
   Solidago canadensis L. var. salebrosa (Piper) M.E. Jones
   Solidago canadensis L. ssp. elongata (Nutt.) Keck
   Solidago canadensis L. var. elongata (Nutt.) M.E. Peck
   Solidago canadensis L. ssp. salebrosa (Piper) Keck
   Solidago dumetorum Lunell
   Solidago elongata Nutt.
   Solidago lepida DC. var. elongata (Nutt.) Fern.
   Solidago lepida DC. var. fallax Fern.
   Solidago canadensis L. var. scabra Torr. & Gray.
   Solidago altissima L.
   Solidago altissima L. var. pluricephala M.C. Johnston
   Solidago altissima L. var. procera (Ait.) Fern.
   Solidago hirsutissima P. Mill.
   Solidago lunellii Rydb.
   Solidago cutleri Fern.
   Solidago deamii Fern.
   Solidago discoidea Ell.
   Solidago xerskinei Boivin [canadensis × sempervirens]
   Solidago fistulosa P. Mill.
   Solidago flaccidifolia Small
   Solidago graminifolia (L.) Salisb.
   Solidago graminifolia (L.) Salisb. var. major (Michx.) Fern.
   Solidago × hirtipes Fern.
   Solidago graminifolia (L.) Salisb. var. nuttallii (Greene) Fern.
   Solidago graminifolia (L.) Salisb. var. polycephala (Fern.) Fern.
   Solidago hirtella (Greene) Bush
   Solidago nuttallii (Greene) Bush
   Solidago polycephala Fern.
   Solidago camporum (Greene) A. Nels.
   Solidago chrysothamnoides (Greene) Bush
   Solidago graminifolia (L.) Salisb. var. gymnospermoides (Greene) Croat
   Solidago graminifolia (L.) Salisb. var. media (Greene) S.K. Harris
   Solidago gymnospermoides (Greene) Fern.
   Solidago gymnospermoides (Greene) Fern. var. callosa S.K. Harris
   Solidago media (Greene) Bush
   Solidago moseleyi Fern.
   Solidago perglabra Friesner
   Solidago texensis Friesner
   Solidago leptocephala Torr. & Gray
   Solidago occidentalis (Nutt.) Torr. & Gray
   Solidago galetorum (Greene) Friesner
   Solidago graminifolia (L.) Salisb. var. galetorum (Greene) House
   Solidago tenuifolia Pursh var. pycnocephala Fern.
   Solidago caroliniana B.S:P.
   Solidago minor (Michx.) Fern.
   Solidago microphylla (Greene) Bush
   Solidago microcephala (Greene) Bush
   Solidago remota (Greene) Friesner
   Solidago tenuifolia Pursh
   Solidago sarothrae Pursh
   Solidago ptarmicoides (Nees) Boivin
   Solidago xbernardii Boivin
   Solidago houghtonii Torr. & Gray ex Gray
   Solidago xkrotkovii Boivin
   Solidago xlutescens (Lindl. ex DC.) Boivin
   Solidago nitida Torr. & Gray
   Solidago ohioensis Frank ex Riddell
   Solidago riddellii Frank ex Riddell
   Solidago corymbosa EII.
   Solidago jacksonii (Kuntze) Fern.
   Solidago rigida L. var. glabrata E.L. Braun
   Solidago rigida L. ssp. glabrata (E.L. Braun) Heard & Semple
   Solidago rigida L. var. laevicaulis Shinners
   Solidago canescens (Rydb.) Friesner
   Solidago jacksonii (Kuntze) Fern. var. humilis (Porter) Beaudry
   Solidago parvirigida Beaudry
   Solidago rigida L. var. humilis Porter
   Solidago rigida L. ssp. humilis (Porter) Heard & Semple
   Solidago grandiflora Raf.
   Solidago rigida
   Solidago parryi (Gray) Greene
   Solidago graminea (Woot. & Standl.) Blake
   Solidago petradoria Blake
   Solidago L.
   Solidago albopilosa E.L. Braun
   Solidago altiplanities C.& J. Taylor
   Solidago puberula Nutt. var. pulverulenta (Nutt.) Chapman
   Solidago pulverulenta Nutt.
   Solidago pulchra Small
   Solidago radula Nutt.
   Solidago radula Nutt. var. laeta (Greene) Fern.
   Solidago radula Nutt. var. radula
   Solidago pendula Small
   Solidago rotundifolia DC.
   Solidago scaberrima Torr. & Gray
   Solidago radula Nutt. Var. stenolepis Fern.
   Solidago roanensis Porter
   Solidago maxonii Pollard
   Solidago roanensis Porter var. monticola (Torr. & Gray) Fern.
   Solidago rugosa P. Mill.
   Solidago rugosa P. Mill. ssp. aspera (Ait.) Cronq.
   Solidago aspera Ait.
   Solidago celtidifolia Small
   Solidago drummondii Torr. & Gray
   Solidago rugosa P. Mill. var. celtidifolia (Small) Fern.
   Solidago rugosa P. Mill. ssp. rugosa
   Solidago rugosa P. Mill. ssp. rugosa var. rugosa
   Solidago scabra Muhl. ex Wilid., non Muhl.
   Solidago rugosa P. Mill. ssp. rugosa var. sphagnophila Graves
   Solidago aestivalis Bickn.
   Solidago rugosa P. Mill. ssp. rugosa var. villosa (Pursh) Fern.
   Solidago rupestris Raf.
   Solidago canadensis L. var. rupestris (Raf.) Porter
   Solidago sciaphila Steele
   Solidago sempervirens L.
   Solidago sempervirens L. var. mexicana (L.) Fern.
   Solidago angustifolia EII.
   Solidago mexicana L.
   Solidago petiolata auct. non P. Mill.
   Solidago sempervirens L. var. sempervirens
   Solidago shortii Torr. & Gray
   Solidago simplex Kunth
   Solidago simplex Kunth ssp. randii (Porter) Ringius
   Solidago simplex Kunth ssp. randii (Porter) Ringius var. gillmanii (Gray) Ringius
   Solidago gillmanii (Gray) Steele
   Solidago glutinosa Nutt. var. gillmanii (Gray) Cronq.
   Solidago racemosa Greene var. gillmanii (Gray) Fern.
   Solidago spathulata DC. var. gillmanii (Gray) Gleason
   Solidago simplex Kunth ssp. randii (Porter) Ringius var. monticola (Porter) Ringius
   Solidago randii (Porter) Britt. var. monticola (Porter) Fern.
   Solidago simplex Kunth ssp. randii (Porter) Ringius var. ontarioensis (Ringius) Ringius
   Solidago glutinosa Nutt. var. ontarioensis Ringius
   Solidago simplex Kunth ssp. randii (Porter) Ringius var. racemosa (Greene)
   Ringius
   Solidago glutinosa Nutt. var. racemosa (Greene) Cronq.
   Solidago racemosa Greene
   Solidago spathulata DC. var. racemosa (Greene) Gleason
   Solidago simplex Kunth ssp. randii (Porter) Ringius var. randii (Porter) Kartesz & Gandhi
   Solidago glutinosa Nutt. ssp. randii (Porter) Cronq. Solidago randii (Porter) Britt.
   Solidago spathulata DC. ssp. randii (Porter) Gleason
   Solidago simplex Kunth ssp. simplex
   Solidago simplex Kunth ssp. simplex var. nana (Gray) Ringius Solidago bellidifolia Greene
   Solidago decumbens Greene
   Solidago decumbens Greene var. oreophila (Rydb.) Fern.
   Solidago glutinosa Nutt. var. nana (Gray) Cronq.
   Solidago oreophila Rydb.

Thus, a Solidago species the extract of which is used according to the invention is a species belonging to the genus Solidago L. A preferred species is Solidago canadensis.

Under "a part of a plant or herb that has grown above the earth" the leaf and/or stem and/or flowers (inflorescence) of the plant is/are meant. Preferably, the extract is prepared from the end of the plant containing many flowers and some leaves. It is especially preferred to prepare the extract from the flowers of the plant.

The extract is prepared in a manner known *per se.* For this purpose, the part of the plant that has grown above the earth, optionally after drying and size-reducing, is extracted. The extraction is carried out with water or an organic solvent such as an alcohol e.g. ethanol or an aqueous solution of an organic solvent e.g. aqueous ethanol (containing 10-60 % by mass of water) generally at 0-100 °C, preferably at 20-100 °C. During the extraction, in most cases, mixing is applied, however, ultrasonication can be used, too. The extract is separated from the parts of the plant by known methods using e.g. sedimentation, pressing of the parts of the plant, filtration, centrifugation or the combination of the procedures listed. The extract obtained can be used as it is or it can be converted to a liquid composition or pharmaceutical composition such as an aqueous solution or syrup. However, it is preferred to remove the solvent content of the extract for example by evaporation, spray drying or freeze drying (lyophilization), and the solid residue is used as an active agent for the preparation of a composition or a pharmaceutical composition. (In the description and claims, the expression "active agent" is used in this sense and it refers to the solid residue that is dissolved in the extract and can be obtained from the extract of the medicinal herb.) Both the extract and the solid residue obtained from the extract can be characterized by the determination of the flavonoid content. For example, the flavonoid content of the solid residue amounts to 2.7-4.1 g/100 g.

Under a "pharmaceutical composition" a known formulation or dosage form is meant which is conventionally used for the prevention or treatment of diseases and which has either systemic or local action. In general, the pharmaceutical composition is suitable for peroral, parenteral, rectal or transdermal administration or for local treatment. Thus, the pharmaceutical composition of the invention is solid or liquid and contains, in addition to the active agent obtained from the medicinal herb by extraction, optionally one or more pharmaceutical carrier(s). The pharmaceutical composition of the invention contains, in general, 0.1-100 % by mass, preferably 1-50 % by mass, suitably 5-30 % by mass of the active agent. It is to be noted that a 100 % content of active agent is possible only in certain cases e.g. in capsules where dilution is not absolutely necessary. In most dosage forms, carriers i.e. diluents and/or other auxiliary agents are needed for the preparation of the pharmaceutical composition.

The solid pharmaceutical compositions suitable for peroral administration may be powders, capsules, tablets, film-coated tablets, microcapsules, lozenge etc., and can comprise binding agents such as gelatine, sorbitol, poly(vinylpyrrolidone) etc.; filling agents such as lactose, glucose, starch, calcium phosphate etc.; auxiliary substances for tabletting such as magnesium stearate, talc, poly(ethylene glycol), silica etc.; wetting agents such as sodium laurylsulfate etc. as the carrier.

Preferred dosage forms include tablets for either systemic or local treatment. Tablets contain e.g. one or more filling agent(s), binding agent(s), lubricant(s), flavouring agent(s) etc. as the carrier. The filling agent is, preferably, a sugar such as xylitol, mannitol, isomaltol or lactose providing for also a sweet flavour. In general, the filling agent is present in an amount of 30 to 60 % by mass. The filling agent may include a further carbohydrate such as starch or microcrystalline cellulose in an amount of generally 10 to 40 % by mass. In most cases, the binding agent includes poly(vinylpyrrolidone) or hydroxypropyl methylcellulose in an amount of generally 1 to 5 % by mass. Lubricants include e.g. 0.2 to 3 % by mass of magnesium stearate, 1 to 5 % by mass of talc or 0.1 to 2 % by mass of silica. If necessary, the flavouring agent is an artificial sweetener or an aroma substance in an amount of generally 0.01 to 1 % by mass. Artificial sweeteners include e.g. aspartame [N-L-α-aspartyl-L-phenylalanine 1-methyl ester], saccharin sodium [1,2-benzisothiazol-3(2H)-one 1,1-dioxide sodium salt], sodium cyclamate [sodium cyclohexylsulfamate] or acesulfame potassium salt [6-methyl-1,2,3-oxathiazin-4(3H)-one 2,2-dioxide potassium salt], the aroma substance is, usually, mint, menthol etc.

Capsules may contain only the active agent or one or more of the carriers detailed above in relation with the tablets.

A further preferred dosage form includes pharmaceutical compositions in the form of a gel that can be employed locally in the oral cavity. Carriers of the gel comprise, suitably, one or more solvent(s), agent(s) providing for a jelly-like consistency, preserving agent(s), artificial sweetener(s), aroma substances etc. The solvent is, in general, distilled water or demineralized water in an amount of generally 50 to 95 % by mass. Also further solvents including glycerol, ethyl alcohol or propylene glycol can be present in an amount of usually 1 to 20 % by mass. Agent(s) providing for a jelly-like consistency include e.g. hydroxypropyl cellulose, sodium carboxymethyl cellulose, calcium carboxymethyl cellulose, poly(vinylpyrrolidone) or guar gum in an amount of generally 0.5 to 5 % by mass. A preferred agent providing for a jelly-like consistency is sodium carboxymethyl cellulose. The preserving agent is, for example, sorbic acid or methylparaben [methyl p-hydroxybenzoate] in an amount of generally 0.1 to 2 % by mass. The artificial sweeteners and aroma substances are usually the ones listed above in an amount of generally 0.1 to 2 % by mass.

The gel of the invention can be introduced into the periodontal pocket by means of a suitable device such as an injection needle to achieve a valuable local effect.

Lozenges are tablets, pills, microcapsules, dragees, biscuits, wafers etc.that dissolve slowly in the mouth, thus, the active agent is liberated in the oral cavity during a longer period. In general, lozenges contain carbohydrate(s) and gelatin as the carrier, furthermore lubricant(s), artificial sweetener(s) and aroma substances as given in relation to tablets. Lozenges contain essentially conventional ingredients in addition to 1 to 30 % by mass of the active agent.

The liquid pharmaceutical compositions suitable for peroral administration may be solutions, syrups, suspensions or emulsions and can comprise e.g. suspending agents such as gelatine, carboxymethyl cellulose etc.; emulsifiers such as sorbitane monooleate etc.; solvents such as water, oils, glycerol, propylene glycol, ethanol etc.; preservatives such as methyl or propyl p-hydroxybenzoate etc. as the carrier.

Preferred liquid dosage forms suitable for peroral administration include solutions containing, for example, solvent(s), preserving agent(s), sweetener(s), aroma substances etc. as the carrier. Solvents include, in general, distilled or demineralized water usually in an amount of 70 to 95 % by mass, furthermore glycerol, ethyl alcohol or propylene glycol as a cosolvent generally in an amount of 1 to 20 % by mass. Preserving agents, sweeteners and aroma substances include, mainly, the ones listed above. The solution can be administered in the form of a spray, thus, introducing an aerosol into the oral cavity.

Pharmaceutical compositions suitable for parenteral application contain, in general, a sterile solution of the active agent.

Pharmaceutical compositions suitable for local treatment include absorbing or non-absorbing threads or chips impregnated with the active agent. Such threads or chips are placed into the periodontal pockets by the dentist to treat periodontitis. In the periodontal pcket, the active agent is slowly absorbed, optionally together with the thread or chip. Non-absorbing threads or chips are later removed by the dentist.

The dosage forms listed above as well as other dosage forms are known *per* se, see e.g. the manual Remington's Pharmaceutical Sciences, 18th edition, Mack Publishing Co., Easton, USA (1990)

The pharmaceutical composition contains dosage unit, in general. The daily dose can be administered in one or more portions. The actual dosage depends on many factors and is determined by the doctor. In general, a typical dose for adult patients of 70 kg body mass amounts to 0.01 to 10 g, preferably 0.1 to 5 g of active agent, daily.

In general, the pharmaceutical composition is prepared by admixing the active agent to one or more carrier(s) and transforming the mixture obtained into a pharmaceutical composition in a manner known per se. The methods that can be used are known from the literature e.g. the manual Remington's Pharmaceutical Sciences cited above. Of course, as a further possibility, the solid residue obtained from the extract can be directly filled into capsules or the extract itself can be converted to a liquid pharmaceutical composition by the addition of further carriers, if needed.

A preferred pharmaceutical composition of the invention is a unit dosage form primarily for systemic action e.g. a capsule, tablet, film-coated tablet etc.

The effect of an extract of a part of a Solidago species, wherein said part has grown above the earth, on diabetic periodontitis was examined in rat as given below. Diabetes was induced in the animals, thus, producing diabetic periodontitis, then the cervix of tooth was ligated in order to increase diabetic periodontitis and the effect of an extract of Solidago canadensis on the diabetic periodontitis was studied. The gravity of the periodontal disease was evaluated on basis of the quantity of Evans blue dye fixed. The higher amount of dye is present in the gingivomucosal tissue, the graver the diabetic periodontitis is.

Diabetes was induced in Spraque-Dawley rats (Charles River, Hungary) having a body mass of 270-290 g by the intravenous administration of streptozocin [2-deoxy-2-{[(methylnitrosoamino)carbonyl]amino}-D-glucopyranose] in a dose of 60 mg/kg. Examination was carried out 6 weeks after the induction of diabetes. In the test animals blood sugar level was steadily high, above 20 mM/liter, and the classical symptoms of diabetes (polyphagia, polyuria, polydipsia, glucosuria, weight loss) could be observed in each animal. In addition, moderate gingival atrophy was experienced.

Periodontitis that accompanied the diabetes was enhanced according to Lohinai [Lohinai, Z., J. Dent. Res., 82, 987 (2003)]. The rats were divided into two groups each consisting of 7 animals. One of the groups was the test group, while the other one served as a control group. The rats were lightly anaesthetized with pentobarbital sodium [5-ethyl-5-(1-methylbutyl)-2,4,6-(1 H,3H,5H)-pyrimidinetrione sodium salt] administered in a dose of 35 mg/kg. Sterile braided silk thread was placed around the cervix of the first bottom molar on the left side and knotted mesially to produce a ligature. In order to speed up the inflammatory process, the silk thread was previously impregnated with a 1 mg/ml aqueous suspension of Salmonella typhimurium, then dried. After recovery from anaesthesia, the rats were housed in a controlled laboratory environment and provided with rodent chow and tap water ad libitum. The animals of the test group were gavaged with 50 mg/kg of the lyophilized active agent prepared from Solidago canadensis by process A of Example 1 once daily for 8 days. The animals of the control group were treated in the same way with physiological saline. On day 8, one hour after the last treatment the rats were re-anaesthetized as above and a cannula was inserted into the right femoral vein. The rate of inflammation was assessed on basis of the vascular permeability of the gingiva and periodontium by the intravenous administration of 50 mg/kg of Evans blue dye dissolved in physiological saline. Five minutes later another cannula was introduced into the abdominal aorta. After 10 minutes the right atria was cut and the dye remaining in the gingivomucosal capillaries was removed by retrograde intraaortic injection of 40 ml of isotonic saline. Then the mandibula was excised, separated from the surrounding tissues and cut in half in a sagittal plane between the incisors. An about 3 mm thick gingivomucosal tissue stripe from the half of the mesiolingual side of the second molar around the mandibular first molar to the half of the mesiobuccal side of the second molar was excised on both sides from the animals for the determination of the Evans blue content. The extravasated Evans blue from the excised gingivomucosal tissue samples was extracted with 0.5 ml of formamide for 48 hours at room temperature, then spectrophotometric determination was performed at 620 nm. The dye content was expressed in µg and related to 1 g of gingivomucosal tissue at the left and right side, respectively. The average of the results obtained were determined for the test group and the control group. The results obtained are shown in Table 1.

**Table 1**

| Treatment | Evans blue content in µg/g | |
|---|---|---|
| | in the left (ligated) gingival tissue | In the right gingival tissue (without ligature) |
| Control | 563 | 265 |
| 50 mg/kg of active agent obtained from an extract of Solidago canadensis | 372 | 208 |

From Table 1 it can be seen that in the control group in which the diabetic periodontitis was increased by the ligature on the cervix of the first molar on the left side, however, no treatment with the medicinal herb extract tested was carried out, the Evans blue content was as high as 563 µg/g indicating a very serious periodontitis. In the same control group at the first molar on the right side at which only the periodontitis owing to diabetes developed by the treatment with streptozocin occurred, however, again, no treatment with the medicinal herb extract tested was carried out, the Evans blue content was 265 µg/g indicating a slighter diabetic periodontitis.

In the test group, at the first molar on the right side at which the diabetic periodontitis was not increased by a ligature on the cervix, treatment with the medicinal herb extract tested resulted in an about 20 % reduction of the periodontitis that accompanied diabetes relative to the control value. At the same time, at the first molar on the left side, the diabetic periodontitis increased by the ligature was reduced very significantly by the peroral treatment with the medicinal herb extract tested: the Evans blue content of the gingiva was reduced to about 65 % of the control value after treatment for 8 days.

### Toxicity tests

5 male NMRI white mice of 23-25 g body mass were treated, intraperitoneally, with a single 200 mg/kg dose of the lyophilized active agent prepared from Solidago canadensis by process A of Example 1, then the behaviour of the animals was observed for a week. No change in the behaviour or body mass of the animals was experienced. Thus, it can be stated that even an extreme high ip. dose of the active agent obtained from the medicinal herb Solidago canadensis does not induce any acute toxic effect.

Based on the above tests, an extract of a part of the medicinal herb Solidago (genus Solidago L.), wherein said part has grown above the earth, or the solid residue remaining after the removal of the solvent content of the extract as the active agent can be used for the effective treatment of diabetic periodontitis.

Thus, an embodiment of the invention is the use of an extract of a part of a Solidago species, wherein said part has grown above the earth, or the solid residue remaining after the removal of the solvent content of the extract as the active agent for the preparation of a pharmaceutical composition suitable for the treatment of the diabetic periodontitis.

A further embodiment of the invention is the use in a method for the treatment of diabetic periodontitis which comprises administering to a patient suffering from diabetic periodontitis an effective non-toxic dose of an extract of a part of a Solidago species, wherein said part has grown above the earth, or the solid residue remaining after the removal of the solvent content of the extract as the active agent.

The invention is further elucidated by means of the following Examples.

### Example 1

### Preparation of an extract

### Process A

100 g of the dry, finely powdered parts of Solidago canadensis grown over the earth and comprising mainly flowers are extracted with water in a mass ratio of 5:200 at 60 °C under intensive stirring over a water bath. The aqueous extract obtained is filtered, the plant matter is pressed, then the extract is sedimented for 4-8 hours, and filtered again. The dry matter content of the aqueous extract obtained amounts to 6.2-6.9 mg/ml. The water is removed by lyophilization while maintaining the temperature of the tray under -50°C. The dry residue obtained is stored in darkness at room temperature and protected from moisture. The dry matter (i.e. the active agent) has a flavonoid content of 3.1-3.4 g/100 g.

### Process B

100 g of the dry, powdered flowers of Solidago canadensis are extracted with water in a mass ratio of 5:150 by boiling at 100 °C. The aqueous extract obtained is worked up as described under process A. The aqueous extract has a dry matter content of 8.5-9.1 mg/ml. The lyophilized product (i.e. active agent) prepared as given under process A has a flavonoid content of 3.8-4.1 g/100 g.

### Process C

100 g of the dry, powdered parts of Solidago canadensis grown over the earth (i.e. leaf, stem, flowers) are extracted with aqueous ethanol containing 75 % by volume of ethanol in a mass ratio of 5:200 in a cold ultrasonic bath. The extract is filtered and the ethanol is removed by evaporation under reduced pressure. The remaining aqueous phase is dried by lyophilization as described under process A.

### Example 2

### Preparation of capsules

0.6 g portions of the lyophilized active agent prepared according to Example 1, process B are filled into hard gelatin capsules, the capsules are closed, placed into a glass container that is sealed airtightly.

### Example 3

### Preparation of capsules

75 g of the lyophilized active agent prepared according to Example 1, process A and 40 g of carboxymethyl cellulose are homogenized, and the mixture is filled into hard gelatine capsules, the capsules are closed, placed into glass containers that are sealed airtightly. Each capsule contains 75 mg of active agent.

### Example 4

### Preparation of tablet

Tablets are prepared from the following ingredients: lyophilized active agent prepared according to Example 1,

| | |
|---|---|
| process A | 10.0 % by mass, |
| lactose | 42.0 % by mass, |
| microcrystalline cellulose | 39.9 % by mass, |
| polyvinylpyrrolidone | 6.0 % by mass, |
| magnesium stearate | 2.0 % by mass, |
| aspartame | 0.1 % by mass. |

The ingredients are homogenized and the mixture is compressed to tablets. Alternatively, the active agent can be homogenized with the lactose and microcrystalline cellulose, the mixture is wetted with an aqueous solution of polyvinylpyrrolidone, then, the magnesium stearate and aspartame are added to the mixture. Finally, the homogenized mixture is compressed to tablets of 500 mg.

### Example 5

### Preparation of syrup

To 1000 ml of the aqueous extract prepared according to Example 1, process A (dry matter content: 6.2 mg/ml), 20 ml of glycerol, 100 ml of 70 % aqueous sorbitol solution, 0.1 g of aroma substance and 1 g of methyl paraben are added, the mixture is homogenized and filled into bottles of 50 ml.

### Example 6

### Preparation of gel

A gel is prepared from the following ingredients:
lyophilized active agent prepared according to Example 1,

| | |
|---|---|
| process A | 10.0 % by mass, |
| demineralized water | 75.9 % by mass, |
| glycerol | 8.0 % by mass, |
| sodium carboxymethyl cellulose | 5.0 % by mass, |
| methylparaben | 1.0 % by mass, |
| mentha | 0.1 % by mass. |

The active agent, methylparaben and mentha are dissolved in the mixture of the water and glycerol, then sodium carboxymethyl cellulose are admixed to the mixture to obtain the gel that is filled into tubes and used to treat the the periodontal pockets.

Alternatively, the gel can be prepared by the following procedure: the active agent and mentha are dissolved in a portion of the water while to the residual water the glycerol is added. In the warm aqueous glycerol solution the methylparaben is dissolved, then the sodium carboxymethyl cellulose is added, the formed gel is stirred until cold, finally the aqueous solution of the active agent and aroma is admixed.

## Claims

1. Use of an extract of a part of a Solidago species, wherein said part has grown above the earth, or the solid residue remaining after the removal of the solvent content of the extract as the active agent for the preparation of a pharmaceutical composition suitable for the treatment of diabetic periodontitis.

2. A use of Claim 1 in which the pharmaceutical composition is a capsule.

3. A use of Claim 1 in which the pharmaceutical composition is a syrup.

4. A use of Claim 1 in which the pharmaceutical composition is a gel especially for the treatment of the periodontal pockets.

5. A use of Claim 1 in which the pharmaceutical composition is an absorbing chip to be placed into a periodontal pocket.

6. Use of an extract of a part of a Solidago species, wherein said part has grown above the earth, or the solid residue remaining after the removal of the solvent content of the extract as the active agent for the preparation of a medicament for the treatment of diabetic periodontitis.

## Patentansprüche

1. Verwendung eines Extraktes eines Teils von einer Solidago-Spezies, wobei der Teil oberhalb der Erde gewachsen ist, oder des festen Rückstandes, welcher nach der Entfernung des Lösemittelgehalts des Extraktes zurückbleibt, als der Wirkstoff für die Herstellung einer pharmazeutischen Zusammensetzung, die für die Behandlung von diabetischer Periodontitis geeignet ist.

2. Verwendung von Anspruch 1, bei der die pharmazeutische Zusammensetzung eine Kapsel ist.

3. Verwendung von Anspruch 1, bei der die pharmazeutische Zusammensetzung ein Syrup ist.

4. Verwendung von Anspruch 1, bei der die pharmazeutische Zusammensetzung ein Gel ist, insbesondere für die Behandlung der periodontalen Taschen.

5. Verwendung von Anspruch 1, bei der die pharmazeutische Zusammensetzung ein absorbierender Chip ist, der in eine periodontale Tasche zu platzieren ist.

6. Verwendung eines Extraktes eines Teils von einer Solidago-Spezies, wobei der Teil oberhalb der Erde gewachsen ist, oder des festen Rückstandes, welcher nach der Entfernung des Lösemittelgehalts des Extraktes zurückbleibt, als der Wirkstoff für die Herstellung eines Medikamentes für die Behandlung von diabetischer Periodontitis.

## Revendications

1. Utilisation d'un extrait d'une partie d'une espèce Solidago, ladite partie poussant au-dessus de la terre, ou du résidu solide restant après l'enlèvement du solvant contenu dans l'extrait en tant qu'agent actif pour la préparation d'une composition pharmaceutique convenant au traitement d'une parodontite diabétique.

2. Utilisation selon la revendication 1, dans laquelle la composition pharmaceutique est une capsule.

3. Utilisation selon la revendication 1, dans laquelle la composition pharmaceutique est un sirop.

4. Utilisation selon la revendication 1, dans laquelle la composition pharmaceutique est un gel spécialement pour le traitement des poches parodontales.

5. Utilisation selon la revendication 1, dans laquelle la composition pharmaceutique est une puce absorbante devant être placée dans une poche parodontale.

6. Utilisation d'un extrait d'une partie d'une espèce Solidago, ladite partie poussant au-dessus de la terre, ou du résidu solide restant après l'enlèvement du solvant contenu dans l'extrait en tant qu'agent actif pour la préparation d'un médicament pour le traitement d'une parodontite diabétique.
